# EUROPEAN PATENT SPECIFICATION

(11) **EP 1 615 539 B1**
(45) Date of publication and mention of the grant of the patent: **14.05.2008**
(21) Application number: 03760827.0
(22) Date of filing: 16.06.2003
(51) Int. Cl.: A61B 1/00, A61M 3/02

(54) **Disposable intestinal intubator with drain and irrigator**
Wegwerfbarer Darmintubator mit Drain und Irrigator
Dispositif d'intubation intestinal jetable pourvu d'un drain et d'un irrigator

(30) Priority: 21.06.2002 LV 020117
(43) Date of publication of application: 18.01.2006
(73) Proprietor: Matasov, Sergej, 1048 Riga (LV)
(72) Inventor: Matasov, Sergej, 1048 Riga (LV)
(86) International application number: PCT/IB2003/002316
(87) International publication number: WO 2004/000091

(56) References cited:
- EP-A- 0 537 985
- EP-A- 1 036 539
- WO-A-01/00260
- WO-A-01/32239
- WO-A-02/19899
- SU-A- 839 553
- SU-A- 927 254
- SU-A- 1 055 519
- US-A- 586 968
- US-A- 3 911 927
- US-A- 4 842 583
- US-A- 6 007 521

## Description

### BACKGROUND OF THE INVENTION.

### TECHNICAL FIELD.

The invention pertains to medicine and is intended for the guaranteed evacuation of cavities' contents, and of the wounds of organism, In particular for colon decompression.

### BACKGROUND ART.

The sharp problem in abdominal surgery is the treatment and prophylaxis of post-operation complications, concerned with loss of hermeticity of intestinal anastomosises. For example, after the operation of removal of obturating colon tumor, the abundance of intestinal contents and high intraintestinal pressure could become the reason of unhermetization of intestinal sutures and of the following peritonitis, mortality of which reaches 75-90 %.

Toddy for colon unloading surgeons are making colostoma, which causes serious inconveniences to patient and requires an additional operation for its closure. In a number of countries colon unloading during and after operation :is realized by drains in the shape of tubes, having latetal openings. However, because of the large dimension of lateral openings, the drain tube is rapidly clogged up with a dense colon contents. Its ablution is possible only on the short length, nearest to anal channel.

Problem of intestinal drains is organically connected with the problem of those insertion into the large intestine, as well as into the small intestine. Insertion of tubular drain is realized by the palpatory method during the operation. However, the fumbling of drain tube and its pushing through intestine, as well as gathering of intestines on the tube, is very prolonged and traumatical procedure, fraught with paresis or intestines. For facilitation of intubation surgeons prefer resilient drains. Moreover, non-resilient drains are disposed to overbendings, Closing these lumen. However during the post-operation period the protracted pressure of resilient tubular drain on the traumatic intestinal wall could lead to its perforation.

There is known the non-resilient "Intestinal drain" which excludes overbendings and allows the filtration of intestinal contents. It is performed in the shape of metal spiral, enclosed in the knitted sheath (see inventors certificate of USSR Nº 927254 from 14.01.1982). However it is also clogged up with intestinal contents. Besides, the insertion of such a drain into colon by the palpatory method is practically exclude.

For the insertion of the "Intestinal drain" it was combined with the "Intestinal Intubator" (see inventors certificates of USSR Nº 839553 from 20.02.1981, Nº 1055519 from 22.07.1983, Nº 1084028 from 08.12.1983, Nº 1377123 from 01.11.1987, Nº 1049067 from 22.06.1983) which comprises an everting thin-wall tube - the so-called invaginator. Transportation of invaginator together with the drain enclosed in it towards the place of invaginator's evertion is realized by the principle «pull-and-push», in other words, by the synergy of vanguard and rearguard forces. Pulling of the non-everted part of invaginator realizes the invaginator itself during its evertion, but the pushing is realized by intractor - a resilient tube, disposed parallel to the non-everted part of invaginator. The internal end of such parallel intractor has a capture of non-everted part of invaginator, but the external one through the seal in the wall of chamber is brought out of it During the insertion of intractor, the invaginator everts, thus unloading the drain, enclosed therein, on intestinal mucosa.

The exploitation of the aforesaid "Inestinal Intubator with Drain" requires the coordinated actions of an operating surgeon and an assistant, who operates the device. The assistant; under surgeon's command connects intractor with invaginator and by hand moves intractor inside. At that time the operating surgeon directs the everting invaginator into colon curves. The fact of reaching by intractor of invaginator's place of evertion is palpatory fixed by operating surgeon. Then the assistant, under surgeon's command, releases infractor from invaginator and returns for its new portion.

The above-mentioned "Intestinal Intubator with Drain" is taken as the prototype of the present invention. To the defects of the prototype belongs drain's occlusion by intestinal:contents, as well as the inconvenience of its insertion:
■ the surgeon palpates the everting.:invaginator, but, being bind with sterility requirements, can not make its feeding;
■ the assistant by manipulation of intractor, realizes feeding of invaginator, but, being devoided of possibility to palpate the invaginator, inevitably commits errors.

### DISCLOSURE OF INVENTION

The invention pertains to abdominal surgery and pursues the elimination of above-mentioned defects, i.e. the achievement of two aims.
■ ensuring of reliable evacuation of intestinal contents;
■ enabling an opportunity to operating surgeon to realize intestines intubation himself.

Theoretically realization of the first aim is connected with creation of:
- the influx-and-extract system of intestinal contents evacuation;
- the possibility to dilute the intestinal contents;
- the possibility to restore the permeability of the drain's wall and channel.

Practically the first aim is achieved by the fact, that the drain-irrigation System of the device includes a hose, which is made, for example, from polyurethane "Elastollan 1180 A 10". In standard the hose is flattened. After connecting of hose to the negative pressure, its cavity is liquidated completely. After connection of hose to the excess pressure, it inflates and turns into the tube. The resilience of such a tube depends on the pressure of gas and/or liquid, included inside it For restoration of permeability of wall and channel of drain and for dilution of intestinal contents, the hose, located in the drain, has plural punctures or holes of minimal dimension. Such hose with punctures could be located not only in drain, but also in invaginator. In case of using of hose as a tensioning device of drain and simultaneosly as its recanalizator, the wall of hose could be hermetic. For increasing of drain's resiliency during intubation and its following recanalization and irrigation, the hermetic hose could be enclosed in the hose with punctures.

The second aim of invention is achieved by the fact that the claimed intestinal intubator with drain comprises:
- the feeder of invaginator with drain and of intractor, which ensures the rearguard intubating force,
- the invaginator, which repeats the form of the drain enclosed in it, and is made, for example, from the polyurethane "Elastollan 1180 A 10",
- the drain with resilience, ensuring the insertion of invaginator with drain,
- the intractor, sequentially connected with invaginator and drain,
- the flexible anal-sigmoid tubus with obturator.

In the feeder of invaginator with drain and intractor the cylinder-piston" unit and pneumatic capture with cuff are used. Cylinder is made in the branch pipe of reel and communicates with the source of negative or excess pressure. In the cylinder there is placed the carriage, consisting of the hollow piston and tube, interconnected by cuff and its distancer, The tube of carriage has the external seal, fixed in the branch pipe of reel. At feeding of excess pressure into the cavity between the piston and external seal in the branch pipe, the cuff squeezes the invaginator with drain or the intractor and they together with carriage are moving in a distal direction. At feeding into the same cavity of negative pressure, the cuff releases the invaginator with drain or the intractor, and carriage under the action of negative pressure is shifting in the original position. The cuff of the feeder could be made, for example, from polyurethane "Elastollan 1180 A 10".

The drain could be made in the shape of spiral spring, enclosed in the sheath with cells. The resilience, necessary for the feeding and then for the intraction of invaginator with drain, could be achieved by selection of the corresponding spring, as well as by arranging in it of pneumatic or hydraulic tensioning device in the shape of hose.

The intractor could be made in the shape of tube, which is sequentially; i.e. "end to end", connected to the invaginator with drain. The definite resilience of tube could be ensured by definite thickness of its wall and by the material, for example, silicone. The resilience of intractor could decrease toward the distal direction (as at the twig).

The flexible anal-sigmoid tubus consists of two short sleeves, connected by the flexible tube, its obturator-from the handle and olive, connected by spring.

### BRIEF DESCRIPTION OF DRAWINGS

The means, ensuring intubation of intestines, are illustrated on drawing 1/2, where
- Fig. 1 -: the reel with a branch pipe, a carriage, an anal collector with a connecting pipes as an assembly on the section;
- Fig. 1A, Fig. 1B -: enlarged fragments of Fig. 1;
- Fig. 2 -: the section of the carriage of the feeder under feeding of negative pressure in it;
- Fig. 2A, Fig. 2B -: enlarged fragments of Fig. 2;
- Fig. 3 -: the section of the carriage of the feeder beyond the branch pipe under feeding of excess pressure in it;
- Fig. 3A, Fig. 3B -: enlarged fragments of Fig. 3.

The means, ensuring the reliable evacuation of intestinal contents, are illustrated on the figures on the drawing 2/2, where is represented the condition of drain-irrigation system of the device in the main moments of its work:

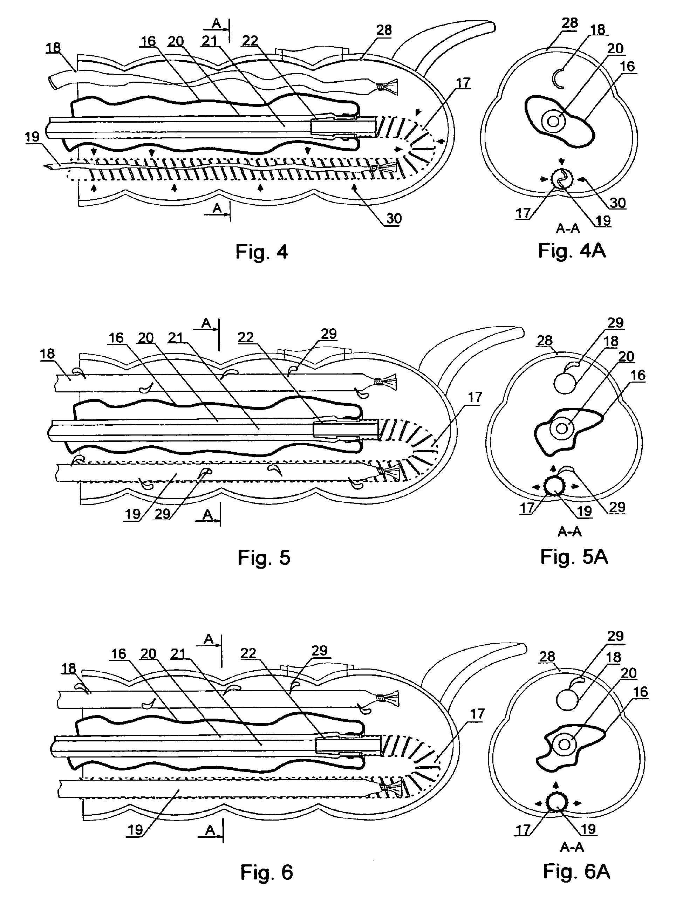

- Fig. 4 -: the system in the "evacuation" mode;
- Fig. 4A -: the A-A section of Fig. 4;
- Fig. 5 -: the system in the "recanalization-irrigation of drain", "irrigation of intestines" mode;
- Fig. 5A -: the A-A section of Fig. 5;
- Fig. 6 -: the system in the "irrigation of intestines", "recanalization of drain" mode at use of the hose with hermetical wall as a tensioning device of invaginator with drain;
- Fig. 6A -: the A-A section of Fig, 6.

### BEST MODE FOR CARRYING OUT THE INVENTION

The description of the intubator with drain and irrigator comprises:
1 - the reel;
2 - the branch pipe;
3 - the seal of tube 12;
4a - the cavity of cylinder, limited by the seals 3 and 9;
4b - the cavity of cylinder, communicated with the everted part 16 of invaginator;
5 - the connecting pipe for feeding of the negative/excess pressure into the cavity 4a;
6 - the distal end of the branch pipe 2;
7 - the connecting pipe for feeding of pressure into the cavity 4b and the averted part 16 of invaginator;
8 - the piston of carriage;
9 - the seal of the piston 8;
10 - the cuff of the carriage;
11 - the cavity of the cuff 10;
12 - the tube of the carriage;
13 - the distancer of the carriage between the piston 8 and the tube 12;
14 - the holes in the distancer 13, which unite its cavity with the cavity of the cylinder 4a;
15 - the uneverted part of invaginator, which includes the drain 17 and the hose 19;
18 - the everted part of invaginator,
17 - the drain;
18 - the hose, located beyond the drain 17;
19 - the hose, enclosed in the drain 17;
20 - the intractor,
21 - the cavity of the intractor 20;
22 - the connecting pipe, uniting cavity 21 of intractor 20 with the cavity of drain 17;
23 - the line, indicating the proximal position of the carriage;
24 - the line, indicating the distal position of the carriage;
25 - the anal collector;
26 - the connecting pipe "evacuation", communicating with cavity of the drain 17;
27 - the connecting pipe of the hose 19;
28 - the cecum;
29 - the small spurts of cleansing agent;
30 - the arrows, indicating the direction of gas or liquid current.

The sterilization of the disposable elements of the device is fulfilled by manufacturer.

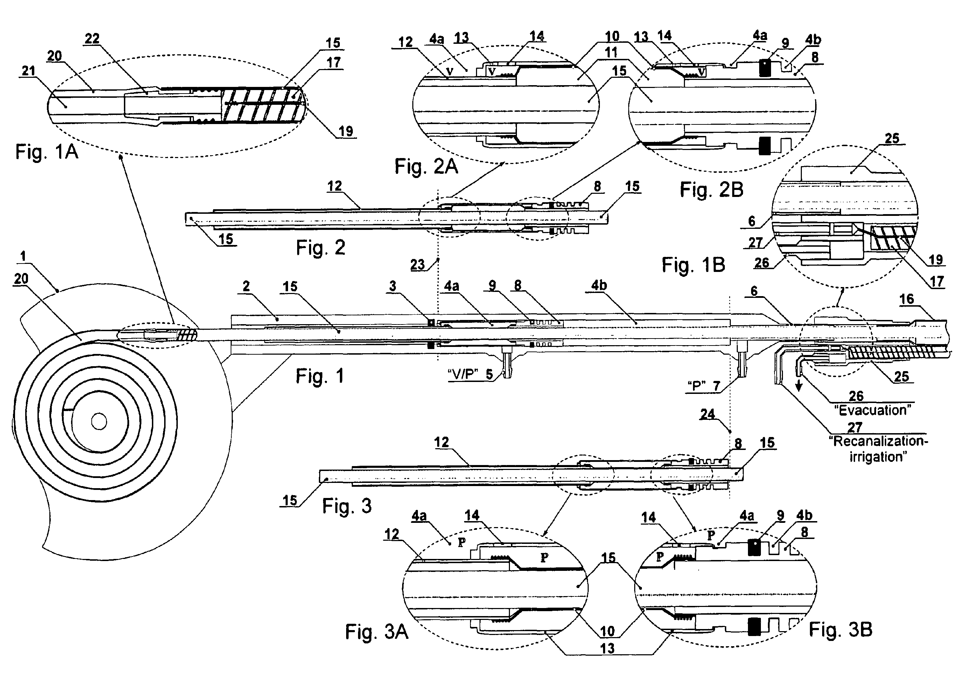

Having removed colon tumour and putted anastomosis, a surgeon makes a resolve about intubation of intestine and then a personnel puts a patient in the position as for the perineal lithotomy, extracts the device illustrated on Fig. 1 from the sterile vacuum packaging and connects it with the sources of the excess and negative pressure. The connecting pipe 5 is joined with the switch "negative/excess" pressure, located on the pedal at the foot of operating surgeon. The connecting pipe 7 is joined with compressor, which creates a working pressure in the cavity 4b communicated with the everted part 16 of invaginator. The connecting pipe 26 is joined to the container of electric aspirator, but the connecting pipe 27 - to the container with cleansing agent.

Then the assistant introduces into the patient's rectum the flexible anal-sigmoida tubus, takes out of it obturator and by the anal collector 25 hermetically connects the distal end 6 of the branch pipe 2 with the anal sleeve of tubus. The preparation for intubation is completed by fastening of the device on the tripod, placing of pedals at the foot of operating surgeon and switching on of the compressor.

The negative pressure, created in cavity 4a, press the cuff 10 to the inner diameter of distancer 13. At pressing on the pedal, negative pressure in the cavity 4a changes on the excess one and cavity 11. disappears, but the cuff 10 hermetically envelopes and squeezes the uneverted part 15 of invaginator with the drain 17, enclosed in it. In such a way the cavity 4b and everted part 16 of invaginator are cutting off from the atmosphere. Due to the compressed cuff 10 and the compressor, permanently connected with connecting pipe 7, in the cavity 4b and in the everted part 16 of invaginator, which continues this cavity, the pressure is created, which everts the invaginator. At the same time the pressure in cavity 4a reaches the level, which ensures the displacement of carriage till the line 24. Due to the evertion, from the invaginator on mucosa the drain 17 and the hose 18 are unloaded. When the distance between lines 23 and 24 is 100 mm, the intestine is intubated on the same distance. The surgeon not only presses on pedal, but also by hands through the wall of intestine directs invaginators evertion in its natural curves.

When the pedal is released, the negative pressure in cavity 4a again presses the cuff 10 to the periphery, releases the uneverted part 15 of invaginator and returns the carriage to the line 23. One intubation cycle include one pressure on pedal and thus the change of the negative pressure on the excess one in the cavity 4a. By the definite number of such a cycles the whole colon is incubated.

A gas, which is feeding in the intestine 28 through the external end of intractor 20 along its channel 21, as well as a weak negative pressure, created through the connecting tube 26 in the channel of drain 17, realize the unloading of colon according to the influx-and-extract principle, which exclude the sticking of drain 17 to mucosa, as well as reduce the probability of fast occlusion of drain 17.

Recanalization and ablution of the wall of drain 17, and then the irrigation of colon is realized through the hose 19 by connecting of liquid pressure to the connecting pipe 27. Firstly the liquid increase the volume of hose 19 up to the inner diameter of drain's 17 spring and through the cells of sheath squeezes out the intestinal contents, which get there. After achieving in the hose 19 of definite pressure, liquid directs to the punctures of its wall and begin to wash the sheath of the drain. Simultaneously liquid arrives in the cavity of intestine and dilute its contents. In case of use of two hoses 19 and 18, the speed of irrigation of intestines increases.

Recanalization-irrigation of drain and irrigation of intestine should be alternated by evacuation of it's contents. For speeding-up of evacuation, the cavity 21 of intractor 20 could be used, the external end of said intractor, as well as connoting tube 26, should be connected to the negative pressure.

## Claims

1. A device for the evacuation of intestinal contents comprising an invaginator everted under the fluid pressure, where the everted part (16) is joined with a branch pipe (2), but the non-everted part (15) repeats the form of a drain (17) enclosed in it along all drain's length, further comprising a hose (19) with punctures enclosed in the drain (17).

2. The device according to claim 1, further comprising a hose (18) with punctures enclosed in the non-everted part (15) of said invaginator.

3. The device according to claim 1 or 2, further comprising an intracter (20), where the end of said intracter is joined with the end of the non-everted part (15) of the invaginator and the drain (17).

4. The device according to any preceeding claim, further comprising a cylinder-piston unit located in the branch pipe (2), where in the cylinder of said unit is placed a carriage consisting of the hollow piston (8) and a tube (12), interconnected by a cuff (10) and its distancer (13) and said tube (12) has an external seal (3) fixed in the branch pipe (2).

## Patentansprüche

1. Eine Vorrichtung für die Evakuation des Darminhaltes bestehend aus einem durch Flüssigkeitsdruck evertiertem Invaginator, dessen evertierter Teil (16) mit einem Verbindungsstück (2) verbunde ist, wohingegen der nicht evertierte Teil (15) in Form und Verlauf der Drainage (17) folgt und in dieser in ganzer Länge eingeschlossen ist, weiterhin umfassend einen perforierten Schlauch (19) innerhalb der Drainage (17).

2. Die Vorrichtung nach Anspruch 1, zusätzlich umfassend einen perforierten Schlauch (18) enthalten im nicht evertierten Teil (15) des Invaginators.

3. Die Vorrichtung nach Anspruch 1 oder 2, zusätzlich umfassend einen Intraktor (20), welcher mit dem Ende des nicht evertierten Teils (15) des Invaginators und der Drainage (17) verbunden ist.

4. Die Vorrichtung nach einem der vorhergehenden Ansprüche, zusätzlich umfassend eine sich in dem Verbindungsstück (2) befindliche Zylinder-Kolben-Einheit, deren Zylinder sich auf einem Schlitten befindet, welcher aus einem hohlen Kolben (8) und einem Rohr (12), welches mit einer Manschette (10) und Ihrer Unterlegscheibe (13) verbunden sind, wobei das Rohr (12) mit der äußeren Dichtung (3) in dem Verbindungsstück fixiert ist.

## Revendications

1. Dispositif destiné à l'évacuation du contenu intestinal, comprenant un invaginateur se retournant vers l'extérieur sous pression de fluide et dont la partie retournée (16) est liée à un tuyau de raccordement (2) et la partie non retournée (15) répète la forme d'un drainage caché (17) à l'intérieur sur toute sa longueur, **caractérisé en ce qu'**il comprend en outre une manche (19) transpercée qui est cachée dans ledit drainage (17).

2. Dispositif selon la revendication 1, **caractérisé en ce qu'**il comprend en outre une manche (18) transpercée qui est cachée dans ladite partie non retournée (15) dudit invaginateur.

3. Dispositif selon la revendication 1 ou 2, **caractérisé en ce qu'**il comprend en outre un intracteur (20) dont l'extrémité est liée à celle de ladite partie non retournée (15) dudit invaginateur et dudit drainage (17).

4. Dispositif, selon l'une quelconque des revendications précédentes, **caractérisé en ce qu'**il comprend en outre une unité cylindre-piston qui est située dans ledit tuyau de raccordement (2), dans le cylindre de laquelle est situé un chariot constitué d'un piston (8) creux et d'un tuyau (12) qui sont reliés par une manchette (10) et son distanceur (13), ledit tuyau (12) ayant une étanchéité extérieure (3) fixée dans ledit tuyau de raccordement (2).
